# EUROPEAN PATENT APPLICATION

(11) **EP 3 257 946 A1**
(43) Date of publication of application: **20.12.2017**
(21) Application number: 15881939.1
(22) Date of filing: 10.02.2015
(51) Int. Cl.: C12P 7/42, A01G 7/06

(54) **METHOD FOR PRODUCING METABOLITE CAPABLE OF IMPROVING DROUGHT TOLERANCE USING MICROORGANISM, AND METHOD FOR IMPROVING DROUGHT TOLERANCE OF PLANT USING SAID METABOLITE**

(71) Applicant: New Environmental Technology Council, Tokyo 102-0094 (JP); Kinki University, Higashiosaka-shi, Osaka 577-0818 (JP)
(72) Inventor: ANO Takashi, Kinokawa-shi Wakayama 649-6493 (JP); HIROSE Yoichiro, Tokyo 105-0012 (JP)
(74) Representative: TBK
(86) International application number: PCT/JP2015/053698
(87) International publication number: WO 2016/129064

(57) **Abstract**

The purpose of the present invention is: to produce a metabolite capable of improving drought tolerance of a plant to the same level as that achieved by abscisic acid, from a microorganism other than a microorganism that is known to be capable of producing abscisic acid; and to provide a method for improving drought tolerance of a plant using the metabolite. A method for producing a metabolite capable of improving drought tolerance using a microorganism, said method comprising culturing a microorganism that belongs to the genus Collimonas and has an ability of producing a metabolite capable of improving drought tolerance of a plant and then extracting the metabolite from a culture produced by the culturing.

## Description

### Technical field

The present invention relates to a method for imparting drought tolerance to plants using a microbial metabolic function.

### Background Art

Abscisic acid is known as one of plant hormones. Known physiological effects of abscisic acid on plants include, for example, a stomatal closure effect, a drought tolerance-improving effect, a seed development and maturation-promoting effect, and a seed dormancy-inducing effect.

Known methods for producing abscisic acid include organic synthesis methods and methods using microbial metabolic functions as disclosed in the patent literatures listed below.

### Citation List

### Patent Literatures

Patent Literature 1: JP 06-197775 A
Patent Literature 2: JP 06-253869 A

### Summary of Invention

### Technical Problem

The patent literatures disclose fungi belonging to the genus Botrytis and the genus Cercospora, as microorganisms having the ability to produce abscisic acid. However, no knowledge has been obtained about other microorganisms, specifically bacteria, having the ability to produce abscisic acid or a metabolite capable of particularly improving drought tolerance as a physiological effect of abscisic acid on plants.

It is therefore an object of the present invention to obtain, from a microorganism other than those conventionally known to have the ability to produce abscisic acid, a metabolite capable of improving drought tolerance of plants similarly to abscisic acid and to provide a method for improving drought tolerance of plants using such a metabolite.

### Solution to Problem

In order to solve the above problem, the inventors have conducted intensive studies focusing on a bacterium belonging to the genus Collimonas, and have arrived at the present invention after have found that the bacterium has the ability to produce a substance capable of imparting drought tolerance to a plant similarly to abscisic acid.

A first aspect of the present invention is directed to a method for producing a drought tolerance improving metabolite using a microorganism, the method including: culturing a microorganism belonging to the genus Collimonas and having the ability to produce a metabolite capable of improving drought tolerance of a plant; and extracting the metabolite from a culture obtained by the culturing.

A second aspect of the present invention is directed to a method for improving drought tolerance of a plant, the method including: culturing a microorganism belonging to the genus Collimonas and having the ability to produce a metabolite capable of improving drought tolerance of a plant; and allowing a culture obtained by the culturing to act on a plant.

A third aspect of the present invention is directed to a method for improving drought tolerance of a plant, the method including allowing the drought tolerance improving metabolite extracted by the method according to the first aspect of the present invention to act on a plant.

### Advantageous Effects of Invention

The present invention makes it possible to obtain, from a microorganism other than those conventionally known to have the ability to produce abscisic acid, a substance capable of imparting drought tolerance to plants similarly to abscisic acid, and also makes it possible to provide a method for improving drought tolerance of plants using such a substance.

### Brief Description of Drawings

FIGS. 1(a) to 1(g) are pictures showing how the tolerance of white radish seeds to a drought environment is improved during germination and the following growth process in test examples, in which FIGS. 1 (a) to 1 (c) show cases where a drought tolerance improving metabolite producing bacterium extract is used, FIGS. 1 (d) to 1 (f) show cases where abscisic acid is used, and FIG. 1(g) shows a case where dH₂O is used as a control.
FIG. 2 is a graph showing the results of a drought tolerance test performed on white radish seeds in test examples.

### Description of Embodiments

Hereinafter, an embodiment of the present invention will be described with reference to the accompanying drawings.

### (Microorganism to be used)

The microorganism used in the method of the present invention for producing a metabolite capable of improving drought tolerance of plants is a Collimonas bacterium. Collimonas sp. NITEP-1104 was used as a specific example of the bacterium. This microorganism has been deposited on June 9, 2011 in NITE Patent Microorganisms Depositary (NPMD) (2-5-8 Kazusakamatari, Kisarazu-shi, Chiba, Japan), and its microbiological properties are disclosed in International Publication WO 2014-141362 A1.

Hereinafter, in the description, the bacterium of accession number NITEP-1104 will be referred to as the drought tolerance improving metabolite producing bacterium.

### (Culture of drought tolerance improving metabolite producing bacterium and extraction of drought tolerance improving metabolite)

Culture of the drought tolerance improving metabolite producing bacterium and extraction of the drought tolerance improving metabolite can be performed using a conventionally known culture method and a conventionally known method for purifying organic compounds, respectively.

For example, the drought tolerance improving metabolite producing bacterium is subjected to liquid culture. The resulting liquid culture is adjusted to pH 2.5 with HCl and then centrifuged. After the centrifugation, the supernatant of the liquid culture is transferred to a separatory funnel and then extracted with ethyl acetate, so that a drought tolerance improving metabolite producing bacterium extract is obtained. After the extraction, the extract is filtered and then concentrated under reduced pressure to dryness using a rotary evaporator and a centrifugal evaporator. In this way, the drought tolerance improving metabolite is successfully obtained.

### (Test Examples)

### Collimonas culture and drought test on white radish using drought tolerance improving metabolite extracted from Collimonas culture

### (1)-1. Test method 1

Samples A, B, and C were prepared containing, respectively, 10, 100, and 300 µg/mL·agar of the drought tolerance improving metabolite producing bacterium extract obtained by the method described above. The samples were each dispensed into 10 test tubes.

ABA standard samples D, E, and F were prepared containing, respectively, 1, 10, and 20 µg/mL·agar of abscisic acid (ABA) as a standard. The samples were each dispensed into 10 test tubes.

As a control, dH₂O sample G was prepared and then dispensed into 10 test tubes.

After a culture medium was prepared in each sample, one white radish seed was placed in each test tube. After each test tube was capped with aluminum foil, each seed was germinated in the dark.

### (1)-2 . Test results

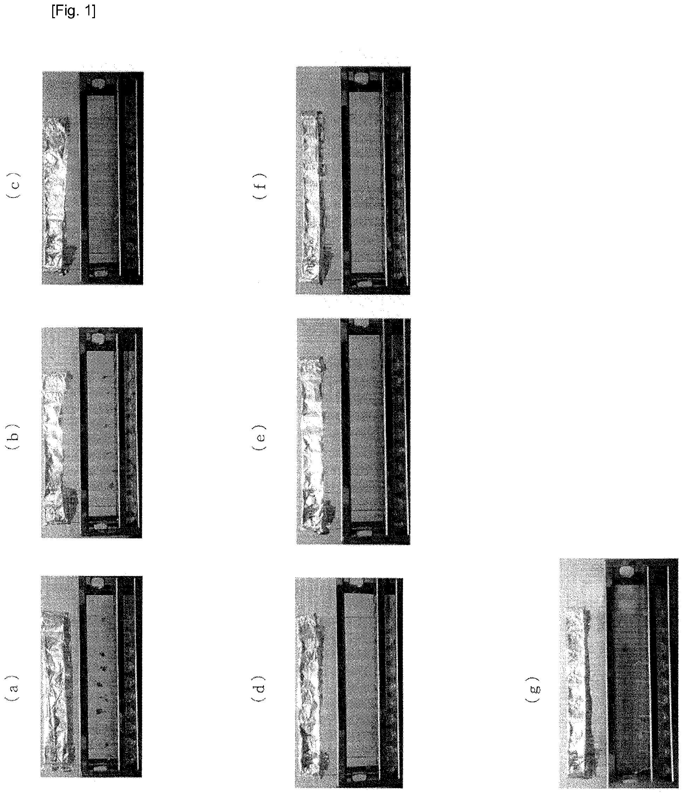

FIGS. 1 (a) to 1 (g) show how the white radish seeds germinate in each sample after 3 days. Strong germination inhibition was observed in ABA standard samples D to F.

Concerning samples A to C containing the drought tolerance improving metabolite producing bacterium extract, growth delay after germination was observed in sample B (100 µg/mL), and weak germination inhibition was observed in sample C (300 µg/mL).

### (2)-1. Test method 2

Samples A to C, D and G prepared in test method 1 described above were subjected to a drought tolerance test after the aluminum foil was removed from each test tube.

### (2)-2. Test results

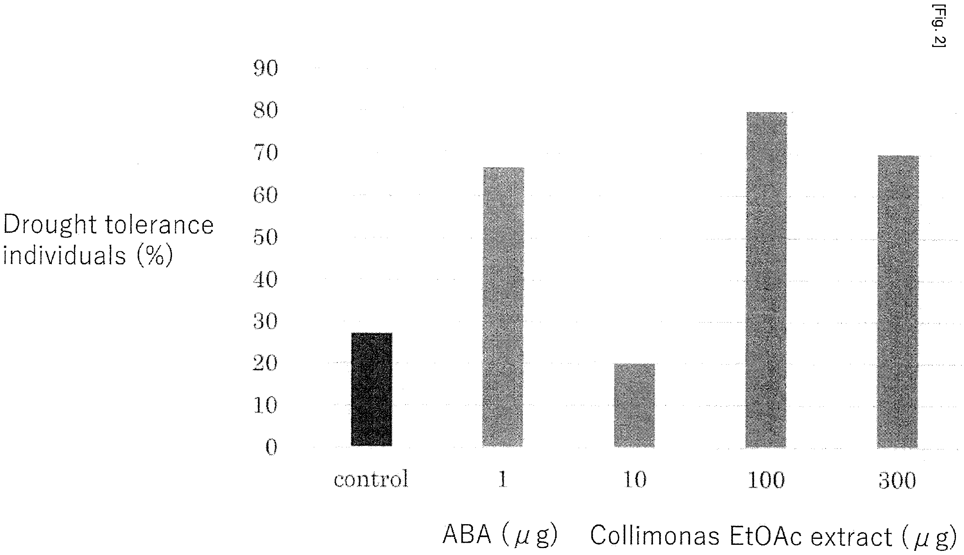

FIG. 2 is a graph showing the drought tolerance level of each sample. The resulting percentage of sound individuals was as follows.

Sample A: 20%
Sample B: 80%
Sample C: 70%
Sample D: 66%
Sample G: 27%

The results suggest that about 100 µg/mL of the drought tolerance improving metabolite producing bacterium extract can impart drought tolerance to plant seeds.

In addition, the results suggest that the resulting Collimonas bacterium culture should contain a plant hormone capable of improving drought tolerance of plants at least similarly to ABA.

## Claims

1. A method for producing a drought tolerance improving metabolite using a microorganism, the method comprising:
culturing a microorganism belonging to a genus Collimonas and having an ability to produce a metabolite capable of improving drought tolerance of a plant; and extracting the metabolite from a culture obtained by the culturing.

2. A method for improving drought tolerance of a plant, the method comprising: culturing a microorganism belonging to a genus Collimonas and having an ability to produce a metabolite capable of improving drought tolerance of a plant; and allowing a culture obtained by the culturing to act on a plant.

3. A method for improving drought tolerance of a plant, the method comprising allowing the drought tolerance improving metabolite extracted by the method according to claim 1 to act on a plant.
